# EUROPEAN PATENT SPECIFICATION

(11) **EP 3 312 268 B1**
(45) Date of publication and mention of the grant of the patent: **18.08.2021**
(21) Application number: 16814296.6
(22) Date of filing: 20.06.2016
(51) Int. Cl.: C12N 5/00, C12M 1/00, C12M 3/00, B01L 3/00, C12M 1/02, C12M 1/34, C12M 1/42

(54) **CELL CULTURE METHOD, USE OF AN APPARATUS FOR CELL CULTURE, AND CELL CULTURE DEVICE**
ZELLKULTURVERFAHREN, VERWENDUNG EINES GERÄTS FÜR ZELLKULTUR UND ZELLKULTURVORRICHTUNG
PROCÉDÉ DE CULTURE DE CELLULES, UTILISATION D'UN RECIPIENT POUR CULTURE CELLULAIRE ET DISPOSITIF DE CULTURE CELLULAIRE

(30) Priority: 22.06.2015 JP 2015124785
(43) Date of publication of application: 25.04.2018
(73) Proprietor: Toyo Seikan Group Holdings, Ltd., Shinagawa-ku Tokyo 141-8627 (JP)
(72) Inventor: SUENAGA, Ryo, Yokohama City Kanagawa 240-0062 (JP); TANAKA, Satoshi, Yokohama City Kanagawa 240-0062 (JP); KASHIWABARA, Ken, Yokohama City Kanagawa 240-0062 (JP); TOTANI, Takahiko, Yokohama City Kanagawa 240-0062 (JP); OTA, Kyohei, Yokohama City Kanagawa 240-0062 (JP)
(74) Representative: Vossius & Partner Patentanwälte Rechtsanwälte mbB
(86) International application number: PCT/JP2016/068212
(87) International publication number: WO 2016/208526

(56) References cited:
- WO-A1-2014/045532
- WO-A1-2014/208004
- WO-A1-2015/004862
- JP-A- 2012 239 401
- JP-A- 2015 116 150

## Description

### [Technical Field]

The present invention relates to culturing of cells in a culture vessel having flexibility, and more specifically to a cell culture method, the use of a cell culture apparatus and a cell culture apparatus, which can stably form aggregates of cells on a cell culture surface in a flexible cell culture vessel.

### [Background Art]

In the field of modern therapies led by gene therapy and regenerative therapy, it is practiced to culture desired cells, a desired tissue or the like under an artificial environment. As illustrative cell culture methods at the present time, the following technologies are known.

As a first method, a culture method that uses a well plate can be mentioned as a method suitably usable in laboratories and the like. According to this method, a plate with one or more wells (recessed portions) formed therein is used, and cells and a culture fluid are introduced in the individual well or wells to conduct culturing of the cells. This first method is, however, accompanied by problems that the risk of mixing of foreign matter is high because the well or wells are exposed to the atmosphere and also that the addition to and collection from every well are irksome.

As a second method having improved sealability, on the other hand, there is also such a sealable tray vessel as exemplified in PTL 1. In this method, the upper surface of a tray having recessed portions, in which cells and a culture fluid have been introduced, is sealed with a film. According to this second method, the sealability is enhanced compared with the first method, so that the above-described contamination risk is alleviated.

Even with the second method, however, the contamination risk is considered to be still high because air also flows in and out upon charging and discharging the culture fluid. In addition, the sealable tray vessel has a three-dimensional shape as it is a culture vessel provided with recessed portions, and therefore is bulky and is not considered to be easy in handling.

As described above, many of such current cell culture methods conduct culturing by allowing cells to gather on solid bottoms such as recessed portions or wells. From the rapid progress of regenerative therapy and the like, however, there is an increasing demand for the mass production of cells in recent years.

As a method of mass production that meets such a demand, a method that automatically conducts mass culture of cells in a closed system by using a flexible culture vessel having gas permeability such as that exemplified, for example, in PTL 2. This flexible culture vessel enables to conduct production on a relatively large scale, and therefore permits the mass production of cells. Moreover, the flexible culture vessel is a closed system, and therefore also has a merit that the contamination risk with foreign matter (bacteria, virus and the like) during a culturing period can be reduced. The flexible culture vessel is therefore preferred.

As indicated, for example, in PTL 3, on the other hand, it has been disclosed to the effect that in the culture of stem cells for regenerative therapy such as neural stem cells, cells can be efficiently mass-cultured by forming aggregates of the cells in an initial stage of the culture.

PTL 3 also discloses a technology in which a vessel with cross-sectionally U-shaped, recessed portions, which facilitate the formation of aggregates of cells, formed on one side thereof is used, the aggregates of cells are formed in the recessed portions, and the vessel is then turned upside down to continue the culturing of cells at a cross-sectionally flat surface on an opposite side. PTL 4 discloses a cell culture apparatus comprising: a placement surface for a flexible culture vessel, said placement surface having a hole for observing the content of the vessel and said apparatus having an element to apply a pressure onto the flexible culture vessel after placement of the flexible culture vessel on the placement surface.
[PTL 1]
   JP 5098471B2
[PTL 2]
   JP 5344094B2
[PTL 3]
   JP 4543212B2
[PTL 4] JP 2012239401 A

### [Summary]

### [Technical Problems]

However, the above-described well plate, sealable tray vessel and vessel with cross-sectionally U-shaped, recessed portions all involve problems in that they require skill in handling and the quality of cultured cells varies depending on the level of worker's skill. Further, they are fundamentally extremely inadequate for mass production in that a worker must be involved, and are also accompanied by a significant problem in that the risks of operation errors and contamination are still high.

On the other hand, the use of a culture method that uses a flexible culture vessel can substantially eliminate the above-described risk, but there is still a room for development as to how to conduct the mass culture of stem cells for regenerative therapies such as, for example, neural stem cells.

With a view to resolving the above-described problems, the present invention has as objects thereof the realization of a cell culture method, the use of a cell culture apparatus and a cell culture apparatus, which can culture various kinds of cells economically with high quality and in large quantities by using culture vessels having flexibility.

### [Solution to Problems]

To achieve one of the objects described above, a cell culture method of the present invention is defined by claim 1.

To achieve another one of the objects described above, the use of a cell culture apparatus of the present invention is defined by claim 3.

To achieve a further one of the objects described above, a cell culture apparatus of the present invention is defined by claim 4.

### [Advantageous Effect of Invention]

According to the present invention, it is possible to mass-culture cells of reduced contamination risk and high quality by using a culture vessel having flexibility while enabling to form aggregates of cells as needed.

### [Brief Description of Drawings]

[FIG. 1]

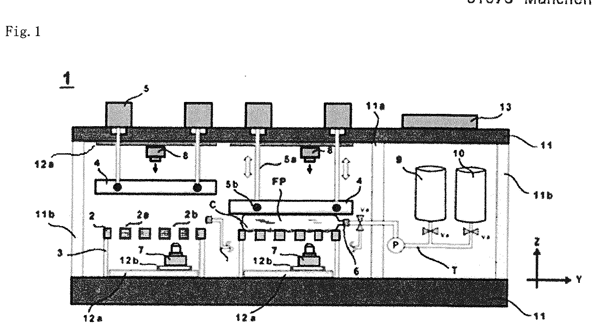

   FIG. 1 is a front view of a cell culture apparatus according to a first embodiment.
[FIG. 2]

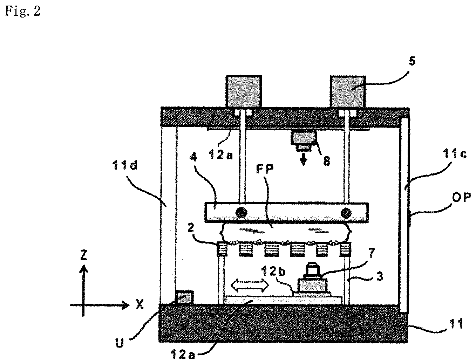

   FIG. 2 is a side view of the cell culture apparatus according to the first embodiment.
[FIG. 3]

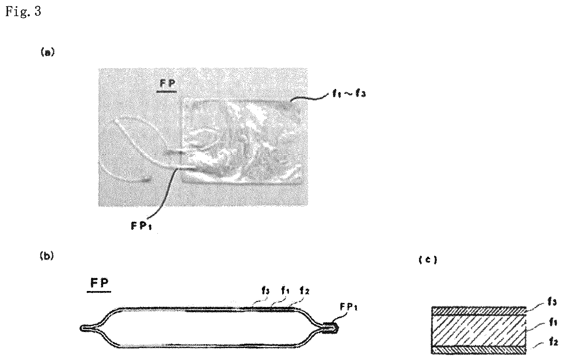

   FIG. 3 depicts views illustrating a flexible culture vessel for use in the first embodiment.
[FIG. 4]

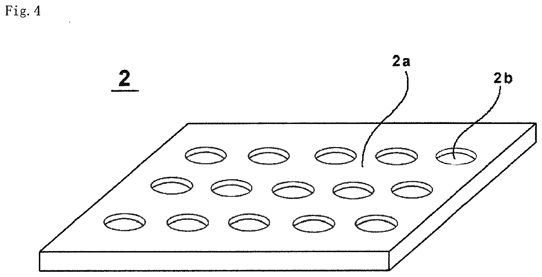

   FIG. 4 is a perspective view depicting details of a stage in the cell culture apparatus according to the first embodiment.
[FIG. 5]

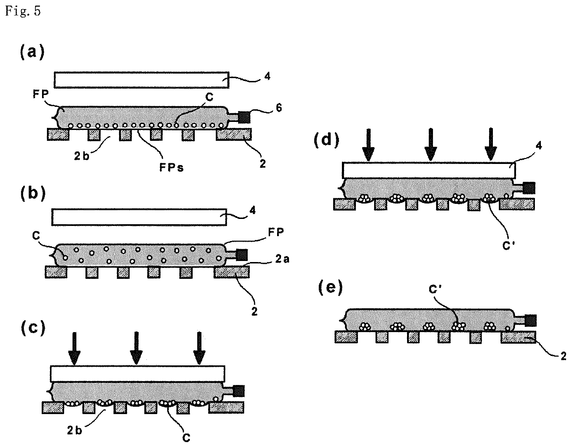

   FIG. 5 depicts flow diagrams illustrating a cell culture method according to the first embodiment.
[FIG. 6]

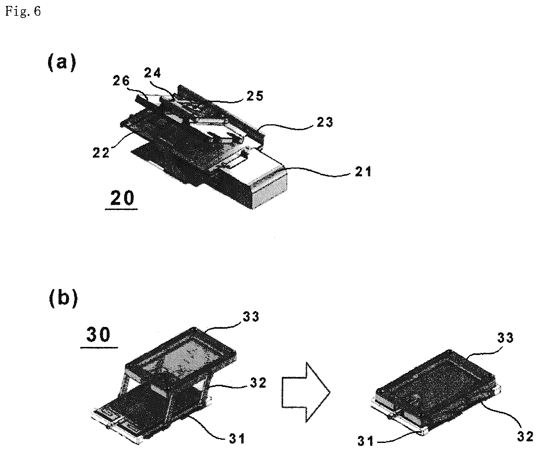

   FIG. 6 depicts perspective views illustrating cell culture jigs applicable in the first embodiment.
[FIG. 7]

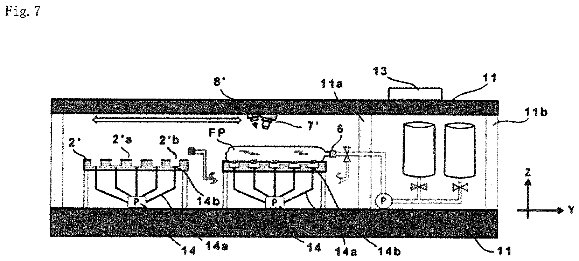

   FIG. 7 is a front view of a cell culture apparatus according to a second embodiment.
[FIG. 8]

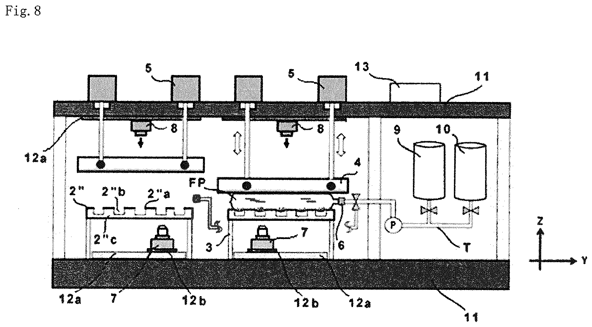

   FIG. 8 is a front view of a cell culture apparatus according to a third embodiment.
[FIG. 9]

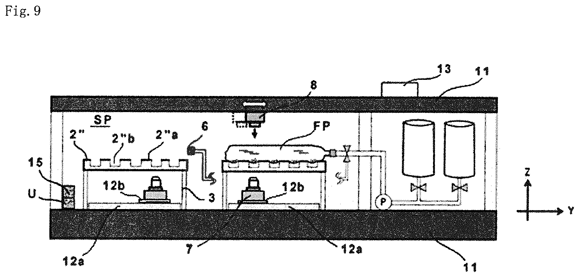

   FIG. 9 is a front view of a cell culture apparatus according to a fourth embodiment.
[FIG. 10]

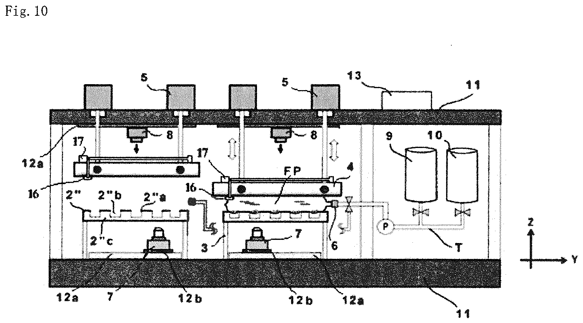

   FIG. 10 is a front view of a mixing unit for a culture fluid and a cell culture apparatus in a fifth embodiment which is not part of the present invention.
[FIG. 11]

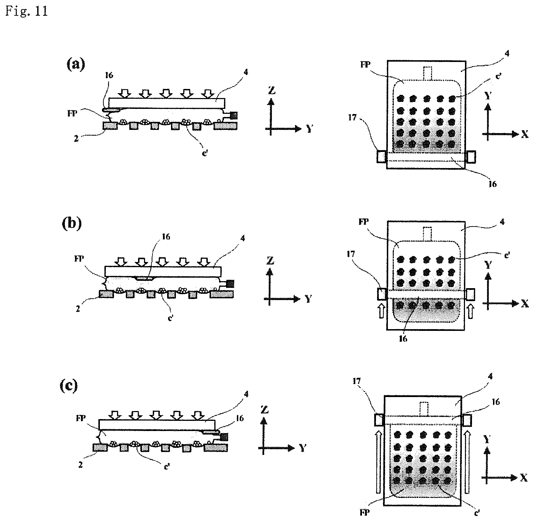

   FIG. 11 depicts state transition diagrams illustrating a mixing method in the fifth embodiment which is not part of the present invention.
[FIG. 12]

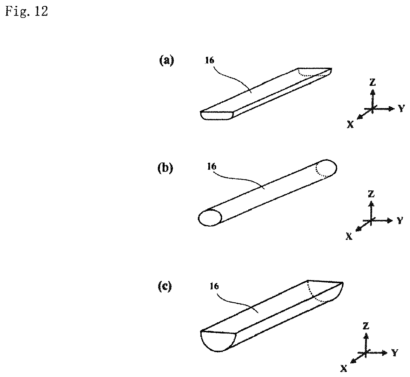

   FIG. 12 depicts modifications of a mixing member 16 in the fifth embodiment which is not part of the present invention.
[FIG. 13]

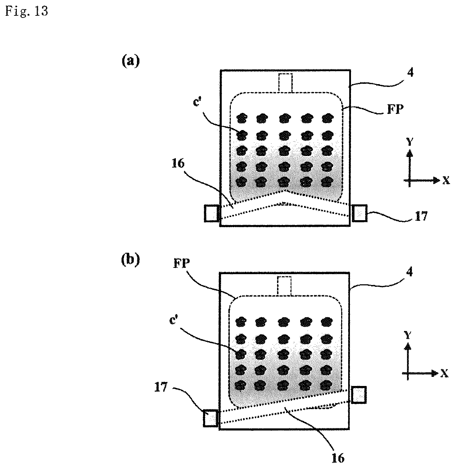

   FIG. 13 depicts views illustrating other modifications of the mixing member 16 in the fifth embodiment which is not part of the present invention.
[FIG. 14]

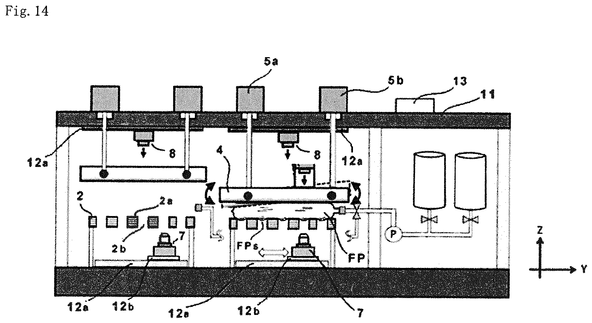

   FIG. 14 is a front view of a cell culture apparatus according to modification 1.
[FIG. 15]

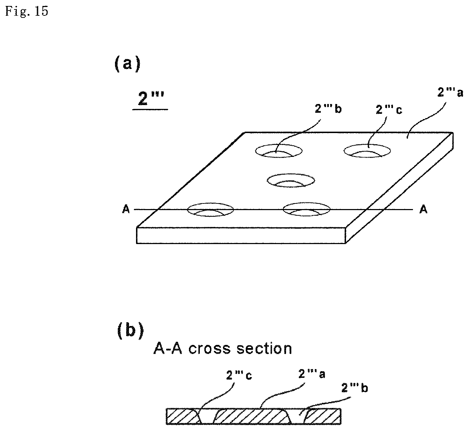

   FIG. 15 depicts a perspective view and a cross-sectional view, which illustrate a stage in a cell culture apparatus according to modification 2.

### [Description of Embodiments]

Referring to the drawings as needed, a description will hereinafter be made specifically about cell culture methods, the use of cell culture apparatus and cell culture apparatus of the present invention. For the sake of convenience of the description, an X-direction, Y-direction and Z-direction will be individually defined, in the following description, but are not intended to limit or restrict the scope of the right of the present invention.

### <<First Embodiment>>

Firstly, a first embodiment of the present invention will be described with reference to FIGS. 1 through 6.

### [Cell Culture Apparatus]

FIG. 1 is a front view of a cell culture apparatus 1 of the first embodiment of the present invention, and FIG. 2 is a side view of the cell culture apparatus 1.

The cell culture apparatus 1 includes at least a stage 2 and a control unit 13. The stage 2 has a placement surface 2a in which one or more depressions 2b are formed. The control unit 13 is programmed to perform control so that subsequent to placement of a flexible culture vessel FP on the placement surface 2a, a pressure is applied onto the flexible culture vessel FP to form recessed and projected portions, which conform to the depressions 2b, in and on an outer surface FPs. The cell culture apparatus 1 is used to culture cells C, which are filled in the flexible culture vessel FP, in a frame 11 controlled at an appropriate temperature (for example, 37°C), carbon dioxide gas concentration (for example, 5% to 10% CO₂ concentration) and humidity (for example, approximately 95%).

The term "flexible culture vessel FP" as used herein means a culture vessel, which is formed in the form of a pouch by using, as a material, a film-based, soft packing material and has flexibility. The flexible culture vessel FP has gas permeability suitable for the culture of the cells C, and preferably has transparency at a part or the entire part thereof to permit visual examination of its contents.

As depicted by way of example in FIGS. 3(a) to 3(c), the flexible culture vessel FP is formed of a multilayer film having a triple layer structure of a base layer f₁, an inner layer f₂, and an outer layer f₃, and is provided with one or more ports FP₁ to perform charging and discharging of a known culture fluid and the cells C.

The base layer f₁ and the inner layer f₂ are formed with a material provided with high gas permeability, heat sealability and transparency. On the other side, the inner layer f₂ is formed with a material provided with low cytotoxicity in addition to the properties described above. As such a material, a polyethylene-based resin such as, for example, linear low density polyethylene (LLDPE), very low density polyethylene (VLDPE) or ultra low density polyethylene (ULDPE), low density polyethylene (LDPE), or a blend of two or more of them can be used.

As the outer layer f₃, on the other hand, a polyethylene-based resin having a density of from 0.886 to 0.93 g/cm³ is suitable. The outer layer f₃ may be omitted as desired.

For the more detailed structure of the flexible culture vessel FP suited for the present embodiment, a reference may be made, for example, to JP 5344094B2 and the like.

No particular limitation is imposed on the cells C to be cultured in the flexible culture vessel FP, but cells for the culture of which the formation of cell aggregates is effective (induced pluripotent stem cells (iPS cells), embryonic stem cells (ES cells), neural stem cells, hepatocytes, corneal stem cells, islet cells, and so on) are particularly suited.

Further, the culture fluid as a medium to be supplied to the flexible culture vessel FP can be also selected, as desired, depending on the cells C to be cultured.

Concerning the cell culture apparatus 1 in which the culture of the cells C is performed using the flexible culture vessel FP with such cells C and culture fluid filled therein, details of individual elements which are also included therein will be described hereinafter.

The stage 2 is a plate member on which the flexible culture vessel FP to be described subsequently herein is to be placed, and is formed, for example, from a metal, a hard resin or the like. In FIG. 1 and some other drawings, the stage 2 is depicted in cross-section to facilitate the understanding of its structure, but actually, it has a plate-like external shape. As depicted in FIG. 4, the stage 2 includes the placement surface 2a, on which the flexible culture vessel FP is placed, and the one or plural depressions 2b formed in the placement surface 2a. In addition, the stage 2b may be also provided, for example, with a fixture or the like for fixedly securing the flexible culture vessel FP on the stage 2. Further, the stage 2 may also be provided with a temperature control unit including, for example, a coil wire, a coil wire with a thermocouple or a Peltier device, so that the temperature of the flexible culture vessel FP can be controlled by the temperature control unit mounted on the stage 2. Furthermore, the stage 2 may also be provided, for example, with a vibration motor, an oscillator or the like as a mechanism for promoting the gathering of the cells C into the depressions 2b and also for mixing the culture fluid. Examples of such an oscillator may include an ultrasonic oscillator, a piezoelectric oscillator, a quartz oscillator, and the like. No particular limitation is imposed on a place where the oscillator is to be arranged, but the oscillator may be arranged, for example, on the placement surface 2a, in each depression 2b, or on a back surface of the stage 2, the back surface being on a side opposite the placement surface 2a.

The placement surface 2a is a planar surface of the stage 2, on which the flexible culture vessel FP is placed. The planar surface has a function to exert a deformation on an outer surface of the flexible culture vessel FP. The plural depressions 2b are formed in the planar surface.

The depressions 2b are locations depressed from the placement surface 2a, and, in the present embodiment, have the shape of a through-hole that extends from the placement surface 2a of the stage 2 to a back surface on the opposite side of the stage 2. Owing to the arrangement of one or the plural number of depressions 2b in the placement surface 2a as described above, one or the like plural number of recesses and projections are formed in and on the placement surface 2a. The "depressions" as described in the present invention are only required to be in such a shape that the outer surface FPs, which is to be described subsequently herein, of the flexible culture vessel FP is formed into a recessed or projected form as a result of an extension of the outer surface FPs from the placement surface 2a into the depressions 2b. The depressions 2b are not necessarily required to extend through the stage 2, and may be formed as recessed portions.

Stage supporting posts 3 are metal or resin members that support the stage 2, and an observation device 7, a stage device 12 and the like can be accommodated in a space inside the stage supporting posts 3. In the present embodiment, the stage supporting posts 3 are disposed in such a way that the stage 2 is supported at four corners thereof by the totally-four stage supporting posts 3.

A pressing member 4 is disposed opposite the flexible culture vessel FP, and is a member of a three-dimensional shape that a bottom surface, which presses the flexible culture vessel FP, is, for example, a planar surface. As the material of the pressing member 4, a transparent resin such as acrylic resin is used in the present embodiment. The shape of the pressing member 4 in plan may desirably be greater than at least the flexible culture vessel FP, but its size or the like is not particularly limited and may be smaller than the flexible culture fluid FP insofar as a desired pressure can be applied to the flexible culture vessel FP. If a lighting device 8 to be described subsequently herein is not needed, the material of the pressing member 4 is not required to be a transparent resin, and the pressing member 4 may be formed, for example, of a metal.

The above-mentioned bottom surface of the pressing member 4 is not absolutely required to be planar. It is possible to use, for example, one having a three-dimensional shape that a bottom surface is a curbed surface (i.e., a surface that is projected in the -Z-direction (downwardly)). In addition, to promote the exchange of gas in the flexible culture vessel FP, one or more holes may be provided through the bottom surface (i.e., the surface on the side opposite the placement surface 2a) of the pressing member 4.

Pressure applying devices 5 are connected to the pressing member 4 via piston rods 5a and connecting links 5b, and cause the pressing member 4 to advance and retract relative to the stage 2 under control of the control unit 13 to be described below. As the pressure applying devices 5 and the piston rods 5a, known piston pumps or the like can be applied.

In the present embodiment, the piston rods 5a are connected approximately to the four corners of the pressing member 4 via the connecting links 5b, whereby the pressing member 4 can be lowered (moved in the -Z-direction) while maintaining the bottom surface (the surface on the side opposite the stage 2) of the pressing member 4 in a horizontal position. As will be described below, the individual piston rods 5a are configured to be independently controllable so that the special orientation of the bottom surface of the pressing member 4 can be adjusted, as needed, during the descend of the piston rods 5a.

The numbers of the piston rods 5a and connecting links 5b for each pressing member 4 are not limited to four described above, and the piston rod(s) 5a and the connecting link(s) 5b may be included in one or more sets. In the drawings, the pressure applying devices 5 are depicted as discrete devices, but a single pressure applying device 5 may be used in common insofar as it can independently control the individual piston rods 5a.

A vessel connection port 6 is disposed for connection with the port FP₁ of the flexible culture vessel FP. In the present embodiment, the opening and closure of the vessel connection port 6 is controlled by the control unit 13. According to this control, a fresh supply of the medium is fed via a tube T to the flexible culture vessel FP placed on the placement surface 2a, and the used medium is collected from the flexible culture vessel FP via the tube T.

In the following, a description will be made taking an example in which a medium supply tank 9 and a medium collection tank 10 to be described subsequently herein are disposed independently of the flexible culture vessel FP, but the first embodiment is not limited to this example. Described specifically, the medium supply tank and the medium collection tank may be individually incorporated as dedicated bags in the flexible culture vessel FP, and the flexible culture vessel FP may be replaced together with the dedicated bags incorporated therein per every cycle of culture of the cells C.

In the present embodiment, the vessel connection port 6 is provided with an identification tag reading part (not depicted) to enable the identification of the contents of the flexible culture vessel FP to be connected to the vessel connection port 6. Described specifically, an identification tag is attached to a part (near the port FP₁ or the like) of the flexible culture vessel FP, and information of the kind of the cells C, the composition of the culture fluid, and the like filled in the vessel is included in the identification tag. As a consequence, the control unit 13 can perform, for example, appropriate culture processing such as the initiation, stop and the like of a culturing operation based on the information read at the identification tag reading part. The term "culturing operation" as used in the present embodiment embraces temperature control and moisture control around the flexible culture vessel FP, operation control of the pressing member 4 (adjustment of the pressing force, and the like), the supply and collection of the medium, and so on. No particular limitation is imposed on the identification tag to be used in the present embodiment, and a known identification member may be used. However, a bar code or quick response (QR) code, a radio-frequency (RF) tag or integrated circuit (IC) tag, or the like is a suitable example.

The observation device 7 is a device that observes the state of the cells C in the flexible culture vessel FA via an object lens or the like, and as the observation device 7, an optical microscope, fluorescence microscope with a charge-coupled device (CCD) and a complementary metal-oxide semiconductor (CMOS) image sensor mounted thereon, or the like can be exemplified. The observation device 7 is arranged below the stage 2 such that the observation device 7 can be moved in horizontal directions on the stage device 12 to be described below. One observation device 7 is arranged for every stage 2 in the present embodiment, but without being limited to such an arrangement, a plurality of observation devices 7 may be arranged for every stage 2 such as an arrangement pattern that a plurality of observation devices 7 are arranged corresponding to the positions of the depressions 2b. Further, the observation device 7 is movable in the horizontal directions, and in addition, is displaceable about the direction of an X-axis (θX-direction) and the direction of a Y-axis (θY-direction), whereby the special orientation of the observation device 7 during an observation can be adjusted. The observation device 7 is configured to permit capturing an image of a region observed as needed, and storing the captured image data in a memory or the like (not depicted).

The lighting device 8 is a device that generates light needed when the observation device 7 observes the state of the cells C in the flexible culture vessel FP, and is arranged in a pair with the observation device 7 in the present embodiment. As the lighting device 8, various known light sources may each be used, but a mercury vapor lamp or the like which generates excitation light may be exemplified, for example, when the observation device 7 is a fluorescence microscope. In the present embodiment, the lighting device 8 is arranged on a side opposite the observation device 7 relative to the stage 2 (placement surface 2a), but without being limited to this arrangement, the lighting device 8 may also be arranged on the same side as the observation device 7 relative to the stage 2 (placement surface 2a). Further, the lighting device 8 may be configured to be movable in horizontal directions while keeping the optical axis of the lighting device 8 in conformity with that of the observation device 7 by a drive mechanism such as an XY stage 12a as depicted in FIG. 1, or as an alternative, an adjustment mechanism may be included to adjust the optical axis of the lighting device 8 relative to the optical axis of the observation device 7. As a further alternative, without moving the lighting device 8, one or more lighting devices 8 may be fixedly arranged on the frame 11 or the like.

The medium supply tank 9 is a receptacle in which a medium (such as a culture fluid) required for the culture of the cells C is stored. The control unit 13 controls a valve Va and a pump P, thereby performing control to supply the medium, which is stored in the medium supply tank 9, to the flexible culture vessel FP via the tube T. A temperature control device (not depicted) is mounted on the medium supply tank 9, and by this temperature control device, the inside of the medium supply tank 9 is kept at a temperature where the medium can be maintained in freshness and quality.

The medium collection tank 10 is a receptacle for collecting the medium the role of which has been completed through its use for the culture of the cells C in the flexible culture vessel FP. The control unit 13 controls another valve Va and the pump P, thereby performing control to collect the unnecessary medium (culture fluid) from the flexible culture vessel FP via the tube T. The medium collected in the medium collection tank 10 may be discarded, or after subjected to an appropriate treatment, may be fed to the medium supply tank 9 for its reuse.

If the medium supply tank 9 and the medium collection tank 10 are in the form of the above-mentioned dedicated bags, on the other hand, pinch valves and a tube roller pump (peristaltic pump) are suited as the valves Va and as the pump P, respectively, so that the culture fluid does not come into contact with the valves Va and the pump P.

The frame 11 is a frame structure forming an accommodation space in which the above-described individual members needed for the culture of the cells C, such as the stage 2 and pressing member 4, are disposed. The frame 11 in the present embodiment is formed of a metal such as stainless steel or aluminum, and as depicted in FIG. 1 and FIG. 2, further includes a partition 11a, a side board 11b, front doors 11c and a rear board 11d. Of these, the partition 11a divides a space, in which the stage 2 and the pressing member 4 are disposed, and another space, in which the medium supply tank 9 and the medium collection tank 10 are disposed, from each other. In the present embodiment, the space in which the flexible culture vessel FP is accommodated can, therefore, be controlled for air conditioning independently from the space in which the medium supply tank 9 and the like are disposed. Further, the front doors 11c are provided with handles OP, so that a worker can open and close the front doors 11c by way of the handles OP in an arbitrary timing.

The stage device 12 includes an XY stage 12a that drives the above-described observation device 7 along two axes in directions parallel to the placement surface 2a under the control of the control unit 13. As the XY stage 12a, it is possible to apply a known two-axis drive mechanism such as, for example, a feed screw mechanism, a linear ball guideway, a cross roller guideway, or a planar motor system.

Further, the stage device 12 further includes a Z stage 12b for adjusting the focal point of the observation device 7. As the focal point may vary depending on the film thickness and the degree of bulges of the above-mentioned recessed portions of the flexible culture vessel FP in the present embodiment, the focal point-adjusting Z stage 12b is particularly effective for realizing high-precision observations. No particular limitation is imposed on the specific adjusting method of the focal point. For example, however, a contrast detection method using image processing may be mentioned as a non-limiting method. The Z stage 12b is disposed below the observation device 7, and moves the observation device 7 in a direction perpendicular to the placement surface 2a (Z-direction). As the Z stage 12b, a desired one of various known mechanisms such as those including one or more screws or air cylinders can be applied. The Z stage 12b may be disposed on the XY stage 12a, or may be moved up and down in the Z-direction together with the XY stage 12a. As a further alternative, the XY stage 12a and the Z stage 12b may be integrated into a stage configuration that enables a drive along at least three axes.

If the lighting device 8 is also arranged below the stage 2 as described above, the lighting device 8 may also be driven along two axes by the stage device 12.

An air-conditioning unit U is arranged on the side of the rear board 11d as depicted in FIG. 2, and is a unit that performs the control of temperature or humidity in the space in which the flexible culture vessel FP and the stage 2 are accommodated. In the present embodiment, the air-conditioning unit U, therefore, performs air-conditioning operation under the control of the control unit 13 so that the temperature and humidity optimal for the culture of the cells C can be maintained.

The control unit 13 is, for example, a computer provided with a memory, in which predetermined programs are stored, and a central processing unit (CPU) having arithmetic functions, and is provided with functions to perform control of the above-described individual members to totally govern the culture of the cells C.

In the drawings, the control unit 13 is arranged on the frame 11 for descriptive purposes, but its arrangement is not limited to this illustrative location. For example, the control unit 13 may be arranged in such a fashion that it is assembled together with a display on the side board 11b, or may be in the form of a personal computer or laptop computer wiredly or wirelessly connected with the above-described individual members as control targets.

### [Cell Culture Method]

With reference to FIG. 5, a description will next be made about a cell culture method of the present embodiment.

Now, the cell culture method of the present embodiment is defined by claim 1 and is primarily characterized by applying a pressure to a culture vessel which is filled with cells and a predetermined culture fluid, has flexibility and is placed on a placement surface having a plurality of depressions, and culturing the cells in the flexible culture vessel with the flexible culture vessel being kept deformed at an outer surface thereof, the outer surface being in contact with the placement surface, under the pressure so applied.

Described specifically, as depicted in FIG. 5(a), the flexible culture vessel FP as a culture vessel is firstly placed on the placement surface 2a of the stage 2 (step 1). The stage 2 includes the depressions 2b formed sunken relative to the placement surface 2a, but in this state, recessed and projected portions conforming to the depressions 2b have not been formed in and on the outer surface FPs of the flexible culture vessel FP.

If the temperature control unit is mounted on the stage 2, the temperature of the culture fluid or the like in the flexible culture vessel FP may be adjusted by controlling the temperature of the stage 2 in step 1 onwards.

As depicted in FIG. 5(b), the cells C in the flexible culture vessel FP are then mixed (suspended) (step 2). By mixing (suspending) the cells C in the flexible culture vessel FP as described above, the number of cells C that will enter the individual recessed portions in a subsequent step can be approximately equalized. The mixing (suspension) processing may preferably be conducted concurrently with the placement of the flexible culture vessel FP on the placement surface 2a or shortly before the pressing by the pressing member 4. However, the timing of the mixing (suspension) processing is not limited to the foregoing, and may be conducted once or a plurality of times from the placement of the flexible culture vessel FP on the placement surface 2a until the initiation of the pressing. If the cells C have already been mixed (suspended) at the time point that the flexible culture vessel FP is placed on the placement surface 2a, step 2 may be skipped.

As depicted in FIG. 5(c), after the flexible culture vessel FP has been placed on the placement surface 2a, the flexible culture vessel FP is pressed with the pressing member 4, whereby recessed and projected portions conforming to the depressions 2b are formed in and on the outer surface FPs of the flexible culture vessel FP (step 3). Described specifically, the control unit 13 of the cell culture apparatus 1 controls the pressure applying devices 5 to move the pressing member 4 downward, and by this movement, the pressing member 4 is pressed against the upper surface (the surface on the side opposite the pressing member 4) of the flexible culture vessel FP. The outer surface FPs of the flexible culture vessel FP is deformed into a shape conforming to the one or plural recesses and projections under the application of the pressure by the pressing member 4.

When the recessed and projected portions have been formed in and on the outer surface FPs of the flexible culture vessel FP by the pressing member 4, the cells C dispersed or suspended in the flexible culture vessel FP begin to gather in the recessed portions (the regions drawn into the depressions 2b). In the present embodiment, the cells C are cultured with the recessed and projected portions, which conform to the depressions 2b in the placement surface 2a, being formed in and on the outer surface of the flexible culture vessel FP as described above. The flexible culture vessel FP is left to stand such that the pressed state of the flexible culture vessel FP by the pressing member 4 continues for a time required to allow all the cells to precipitate, for example, for from several minutes to several tens of minutes. As a consequence, the cells C suspended in the culture fluid have all been allowed to precipitate after the standing.

As depicted in FIG. 5(d), the culture of the cells C is then continued while the pressing of the flexible culture vessel FP by the pressing member 4 is maintained (step 4). In step 4, the above-mentioned aggregation of the cells C in the recessed portions proceeds so that aggregates C' are formed. How big the aggregates C' should be allowed to grow widely varies depending on the kind of the cells C to be cultured, and therefore may be suitably determined through an experiment or simulation.

In step 3 and step 4, the pressing force by the pressing member 4 may be adjusted by changing it based on the state of the outer surface FPs of the flexible culture vessel FP, the degree of aggregation of the cells C, and the like as acquired from the observation device 7. As is understood from the foregoing, the observation device 7 in the present embodiment is configured to permit observations of the inside and outer surface of the flexible culture vessel FP with the pressing by the pressing member 4 being kept applied to the flexible culture vessel FP. Further, the observation device 7 in the present embodiment is configured to permit the observation of the inside of the flexible culture vessel FP via the depressions 2b.

As depicted in FIG. 5(e), after a predetermined time has elapsed since the above-described formation of the aggregates of the cells C in the recessed portions, the recessed and projected portions formed in and on the outer surface FPs of the flexible culture vessel FP are planarized by releasing the pressing by the pressing member 4 during the culture of the cells C (step 5). Described specifically, the control unit 13 of the cell culture apparatus 1 controls the pressure applying devices 5 to move the pressing member 4 upward, and by this movement, the pressing member 4 which has been pressed against the upper surface of the flexible culture vessel FP is allowed to retract upward. As a consequence, the outer surface FPs of the flexible culture vessel FP released from the pressing force, the outer surface FPs being in contact with the placement surface 2a, is brought into a planar form in which the outer surface FPs is substantially parallel with the placement surface 2a, whereby the regions (area) to be used for the culture of the cells can be enlarged.

By leaving the flexible culture vessel FP to stand for the predetermined time in the state of step 5, the culture of the cells proceeds further, and after the elapse of the predetermined time, the culture of the cells ends. Subsequent to the end of the culture of the cells, the worker opens the front doors 11c and takes out the flexible culture vessel FP from the placement surface 2a, whereby the work is completed.

Here, the reason for releasing the applied pressure (pressing force) during the culture of the cells C is as will be described next.

Depending on the kind of the cells C to be cultured, the efficiency of culture is substantially improved when aggregates are formed. If the recessed portions remain, on the other hand, inconvenience can be assumed to arise during the culture.

Described more specifically, it should also be assumed that during the culture of the cells C, a need arises to improve the freshness of the culture fluid in the vessel by replacing a portion of the culture fluid and also to allow gas, which permeates into the vessel (such as carbon dioxide and oxygen), to efficiently diffuse in the vessel. However, if the recessed and projected portions remain in and on the outer surface FPs of the flexible culture vessel FP and especially when the amount of the medium (the culture fluid or the like) is small, no sufficient clearance can be assured to remain between the bottom surface of the vessel and the opposite surface at the projected portions, presumably leading to a potential problem that the circulation of the culture fluid, the diffusion of the gas, and the like may stop.

In the present embodiment, it is, therefore, configured, as depicted in FIG. 5(e), to realize efficient circulation and diffusion of the culture fluid and gas by planarizing the recessed and projected portions of the flexible culture vessel FP after the formation of the aggregates C' of a predetermined size.

If it is desired to realize efficient circulation and diffusion of the culture fluid and gas without using this method, it may be contemplated, for example, to transfer the aggregates C' into a large vessel. This approach is, however, accompanied by various risks as will be described below. If work is performed to transfer the aggregates C' into a vessel having a large area, it is necessary to use a pipette or the like. Various risks, therefore, arise such that the aggregates C' may break up due to strong mixing of the culture fluid and a physical stress such as a shear force may be applied to each cell C to give deleterious effects on the subsequent culture.

However, the use of such a method as in the present embodiment makes it possible to enjoy effects, which are substantially equivalent to those available from the transfer of the aggregates C' to the large vessel, without being accompanied with the above-mentioned risks by simply removing the pressing by the pressing member 4.

During or in parallel with step 1 to step 5 described above, a fresh supply of the medium may be delivered as needed into the vessel from the medium supply tank 9, or the used medium may be collected from the vessel into the medium collection tank 10.

The observations of the cells C, their aggregates and the like by the observation device 7 may be conducted continuously or intermittently during step 1 to step 5. As an alternative, the aggregates and the like at plural different locations among the recessed and projected portions of the flexible culture vessel FP may be sequentially observed by driving the observation device 7 with the stage device 12 as needed.

### [Cell Culture Apparatus]

As the flexible culture vessel FP is reduced in the risk of contamination and its handling is easy as described above, a portable cell culture jig was adopted to hold the flexible culture vessel FP in the present embodiment. An objective of the use of this cell culture jig is to realize the culture of cells in a mode, which is more efficient and is reduced in contamination risk, while retaining the advantage that it can be carried by hands like a conventional flask or well plate.

Described specifically, the use of a cell culture apparatus of the present embodiment is defined by claim 3 and primarily characterized by including a placement surface that exerts a deformation on an outer surface of a flexible culture vessel in which cells and a culture fluid are filled, and a pressing lid disposed opposite the placement surface and capable of moving between a first position, where the flexible culture vessel can be held between the pressing lid and the placement surface while pressing the flexible culture vessel placed on the placement surface, and a second position where at least a part of the pressing lid is more remote relative to the placement surface than at the first position.

According to this cell culture apparatus, it is possible to perform transfers of the flexible culture vessel FP, for example, among a CO₂ incubator that is commonly used in the culture of cells, a clean bench where the addition and replacement of a culture medium are conducted, and a microscope that performs observations of cells in the culture vessel, while holding the flexible culture vessel FP.

Described specifically, as depicted in FIG. 6, one of two kinds of apparatus one being a cell culture apparatus 20 and the other a cell culture apparatus 30, is suitably used in the present embodiment.

As depicted in FIG. 6(a), the cell culture apparatus 20 includes a base 21, a placement plate 22 disposed on the base 21 and having a plurality of depressions, and a pressing lid 26 disposed opposite the placement plate 22.

The pressing lid 26 is connected with an upper lid 24 via bars 25, and can be moved toward or away relative to the upper lid 24 while its spatial orientation is maintained by the bars 25. Also, the upper lid 24 is connected with the base 21 via a hinge 23, and is configured to be turnable about the hinge 23 as an axis.

Therefore, upon accommodating the flexible culture vessel FP in the cell culture jig 20, the flexible culture vessel FP is firstly placed on the placement plate 22 with the upper lid 24 being opened via the hinge 23 (the pressing lid 26 assumes the second position), and the upper lid 24 is then closed until the upper lid 24 is locked relative to the base 21 by a locking mechanism (not depicted) (at least a part of the pressing lid 26 moves toward the placement plate and assumes the first position).

At this time, the pressing lid 26 moves toward the placement plate 22 via the bars 25 along the movement of the upper lid 24, whereby a pressing force is applied by the pressing lid 26 onto the flexible culture vessel FP. Here, the pressing force can be produced by the mass of the pressing lid 26, or can be produced by locking the pressing lid 26 with the locking mechanism (not depicted) with the pressing lid 26 being kept pressed against the flexible culture vessel FP.

The worker then performs the work of culturing the cells in the flexible culture vessel FP by carrying the cell culture apparatus 20, with the pressing force being applied to the flexible culture vessel FP by the pressing lid 26, into the CO₂ incubator by hands or the like.

The cell culture jig 20 of the present embodiment may also be used, for example, in the cell culture apparatus 1 instead of the mode in which it is used in the CO₂ incubator. If the cell culture apparatus 20 is used in the cell culture apparatus 1, the stage 2 may be omitted. In this case, a structure that the base 21 and free ends of the stage supporting posts 3 can be connected and fixed together is preferred.

On the other hand, the cell culture apparatus 30 includes, as depicted in FIG. 6(b), a lower lid 31 having a placement surface with one or more depressions formed therein, a pressing lid 33 disposed opposite the placement surface of the lower lid 31, and parallel links 32 connecting the lower lid 31 and the pressing lid 33 together.

Upon placing the flexible culture vessel FP on the placement surface of the lower lid 31, the pressing lid 33 is moved to the second position as depicted on a left side of FIG. 6(b).

After the flexible culture vessel FP has been placed on the placement surface, the parallel links 32 are driven to press the pressing lid 33 against the flexible culture vessel FP as depicted on a right side of FIG. 6(b).

The lower lid 31 and the pressing lid 33 are next locked together by the locking mechanism (not depicted) while maintaining the pressing force against the flexible culture vessel FP by the pressing lid 33 (the pressing lid 33 moves toward the placement surface and assumes a first position).

A mode in which the flexible culture apparatus 30 is used in the CO₂ incubator or the cell culture apparatus 1 is similar to the above-mentioned case of the cell culture jig 20, and therefore its description is omitted.

In the first embodiment described above, the example in which two stages 2 are disposed side by side in the Y-direction is presented in FIG. 1. The first embodiment is not limited to this example, and only one stage 2 may be disposed or three or more stages 2 may be disposed. In addition, the example in which only one stage is disposed in the X-direction is presented in FIG. 2. The first embodiment is not limited to the example, and two or more stages may be disposed side by side.

Moreover, a plurality of flames 11 in each of which the stage 2 is accommodated may be disposed side by side in a height direction (the Z-direction). As an alternative, the interior of the frame 11 may be divided into plural compartments in the height direction, and the stage 2 and the pressing member 4 may be disposed in each of the compartments so divided.

### <<Second Embodiment>>

Next, a description will be made about a second embodiment of the present invention with reference to FIG. 7.

Differences of the second embodiment from the first embodiment reside, for example, in that a pressure to be applied onto the flexible culture vessel FP is produced by a negative pressure source 14 via a stage 2' and also in that an observation device 7' is arranged above the stage 2'.

These differences from the first embodiment will hereinafter be described primarily, and the same configurations and functions as in the first embodiment will be identified by the same symbols and their description will be omitted unless needed (this will equally apply to third to fifth embodiments and modifications 1 and 2 to be described subsequently herein).

As depicted in FIG. 7, the stage 2' of the cell culture apparatus 1 of the present embodiment is provided with a placement surface 2'a and depressions 2'b sunken downwards (-Z-direction) from the placement surface 2'a. The depressions 2'b in the present embodiment are not formed as through-holes that extend in the Z-direction, but are formed as bottomed portions recessed relative to the placement surface 2'a. Further, flow channels 14b are formed in the bottoms of the depressions 2'b.

In addition, the cell culture apparatus 1 of the present embodiment is provided with a negative pressure generator that produces, via the depressions 2'b, a suction force to the flexible culture vessel FP placed on the placement surface 2'a of the stage 2'. This negative pressure generator includes the negative pressure source 14 and pipings 14a that connects the negative pressure source 14 and the flow channels 14b of the stage 2' together.

As the negative pressure generator is disposed below the stage 2' as described above, the observation device 7' in the present embodiment is arranged above relative to the stage 2' and is configured to be movable in the X-direction or Y-direction. The present embodiment does not include the pressing member 4, so that a lighting device 8' is also arranged side by side with the observation device 7'.

By the second embodiment described above, the outer surface FPs of the flexible culture vessel FP is also formed into a shape conforming to the depressions 2'b a plurality of recessed portions under the application of a negative pressure by drawing the outer surface FPs of the flexible culture vessel FP by using the negative pressure generator without using the pressing member, whereby recessed and projected portions conforming the depressions 2'b of the stage 2' can be also formed in and on the outer surface FPs.

In the present embodiment, the depressions 2'b are internally depressurized using the negative pressure source 14. However, a positive pressure may be applied to the flexible culture vessel FP via the depressions 2'b by using a positive pressure source instead of the negative pressure source.

### <<Third Embodiment>>

Next, a description will be made about a third embodiment of the present invention with reference to FIG. 8.

Differences of the third embodiment from the first embodiment reside, for example, in that a stage 2" is formed of a transparent material such as acrylic resin and also in that depressions 2"b of the stage 2" are bottomed recessed portions rather than through-holes.

The observation device 7 in the present embodiment can observe aggregates or the like of cells C in the flexible culture vessel FP via the transparent stage 2", and moreover can also observe a medium on a placement surface 2"a, suspended cells C and the like as needed.

In other words, the observation device 7 can observe and capture images at all locations of the flexible culture vessel FP in the present embodiment.

In the present embodiment, the depressions 2"b are formed as the bottomed recessed portions rather than through-holes. The present embodiment is not limited to this form, but the depressions 2"b may be formed as through-holes.

According to the third embodiment described above, it is possible to determine whether cells C locally exist at a location or locations other than the recessed portions in the flexible culture vessel FP, whereby the cells C are efficiently allowed to gather in the recessed portions and to form aggregates there.

### <<Fourth Embodiment>>

Next, a description will be made about a fourth embodiment of the present invention with reference to FIG. 9.

Differences of the fourth embodiment from the first embodiment reside, for example, in that a stage 2" is formed of a transparent material such as acrylic resin, in that depressions 2"b of the stage 2" are bottomed recessed portions rather than through-holes, and also in that the pressing member 4 has been omitted and a pressure regulator 15 is included.

Described specifically, as depicted in FIG. 9, the pressure (gas pressure) in a space SP in which the flexible culture vessel FP is disposed is raised by the pressure regulator 15 instead of pressing by the pressing member 4 in the present embodiment.

Since the depressions 2"b in the present embodiment are the recessed portions, on the other hand, the recessed portions exist as closed spaces when the flexible culture vessel FP is placed on a placement surface 2"a.

Therefore, the pressure in the space SP becomes higher than that of the closed spaces upon application of a pressure by the pressure regulator 15, whereby recessed and projected portions conforming to the depressions 2"b are formed in and on the outer surface FPs of the flexible culture vessel FP.

According to the fourth embodiment described above, the flexible culture vessel FP is pressed in a non-contact fashion instead of being physically pressed by the pressing member 4 or the like, so that the desired recessed and projected portions can be formed while inhibiting damage to the flexible culture vessel FP if at all possible.

### <<Fifth Embodiment>>

Next, a description will be made about a fifth embodiment which is not part of the present invention with reference to FIGS. 10 to 13.

Differences of the fifth embodiment from the first embodiment reside, for example, in that the present embodiment includes a mixing unit and a mixing method for a culture fluid and also in that the present embodiment has a mixing member 16 insertable between the flexible culture vessel FP pressed by the pressing member 4 and the pressing member 4, and mixing member driving devices 17 that drive the mixing member 16.

As described above concerning the respective embodiments, the cells C in the culture fluid in the flexible culture vessel FP can exist as the aggregates C'. Here, for the purpose of effectively supplying nutrients to the cells C or a like purpose, an operation is needed to mix the culture fluid in the flexible culture vessel FP at every predetermined time. As the aggregates C' have been formed in the flexible culture vessel FP at this time, it is desired to mix only the culture fluid without causing the aggregates C' to move if at all possible.

The mixing unit and the mixing method to be described below are not part of the present invention.

### <Mixing Unit for Culture Fluid>

As depicted in FIG. 10, the mixing unit of the present embodiment for the culture fluid includes the placement surface that exerts a deformation on the outer surface of the flexible culture vessel FP with cells C and the culture fluid filled therein, the pressing member 4 that applies a pressure onto the flexible culture vessel FP placed on the placement surface, the mixing member 16 insertable between the pressing member 4 and the flexible culture vessel FP, and the mixing member driving devices 17 that drive the mixing member 16.

As the placement surface, it is possible to use the placement surface corresponding to the stage (stage 2, stage 2' or stage 2") disclosed above with respect to any one of the first embodiment to the fourth embodiment.

The mixing member 16 has a function that it is inserted and moved between the pressing member 4 and the flexible culture vessel FP while the state of pressing against the flexible culture vessel FP by the pressing member 4 is maintained. No particular limitation is imposed on the material of the mixing member 16, and a metal material such as stainless steel or a resin material such as plastics can be used, for example. To a surface of the mixing member 16 where it comes into contact with at least the flexible culture vessel FP, desired one of various surface treatment or processing such as, for example, coating with a fluorine-containing material and polishing may be applied to provide the surface with reduced friction. From the viewpoint of reducing the friction caused by contact between the pressing member 4 and the mixing member 16, the above-described surface treatment or processing may also be applied to a surface of the mixing member 16 where the mixing member 16 opposes the pressing member 4.

Described more specifically, as also depicted in FIG. 11, a rectangular parallelepiped, which is rectangular in cross-section and has been chamfered in a round shape at four corners thereof, can be exemplified as the mixing member 16 in the present embodiment. Here, the thickness of the mixing member 16 as measured in the Z-direction may be suitably set, for example, according to the thickness of the flexible culture vessel FP. If the thickness of the mixing member 16 is excessively large relative to the thickness of the flexible culture vessel FP, however, biting of the flexible culture vessel FP and/or movements of the aggregates C may be induced during mixing. Hence, the thickness of the mixing member 16 (as a non-limiting example) may be set preferably at 3 mm or smaller. Further, the width of the mixing member 16 (as a non-limiting example) as measured in the Y-direction may be suitably set according to the width of the flexible culture vessel FP as measured in the Y-direction, and may preferably be, for example, approximately from 5 to 10 mm. Furthermore, the length of the mixing member 16 (as a non-limiting example) as measured in the X-direction may be suitably set, for example, according to the width of the flexible culture vessel FP as measured in the X-direction, and may preferably be greater, for example, than the width of the flexible culture vessel FP as measured in the X-direction.

The mixing member driving devices 17 have functions to support the mixing member 16 and also to allow the mixing member 16 to move in an inserting and moving direction (the Y-direction) between the flexible culture vessel FP and the pressing member 4. As each mixing member driving device 17, desired one of various known power mechanisms can be applied. Illustrative may be a gas-actuated cylinder, a rack and pinion mechanism, a ball screw linear motion mechanism, or a linear motion mechanism which uses magnets.

As depicted in FIG. 10, the mixing member driving devices 17 in the present embodiment are mounted on one side (the upper side) of the pressing member 4, and are connected with the mixing member 16 disposed on the other side (the lower side) of the pressing member 4. As a consequence, the mixing member driving devices 17 can follow the movement of the pressing member 4 even when the pressing member 4 is caused to advance or retract relative to the stage 2 by the pressure applying devices 5.

The mixing member driving devices 17 are not absolutely needed to be mounted on the pressing member 4, and may be disposed, for example, on the side of the placement surface. If the mixing member driving devices 17 are disposed at locations other than the pressing member 4, it is preferred to dispose the mixing member 16 and the mixing member driving devices 17 via resilient members (spring mechanisms or the like) which are displaceable in the Z-direction, so that the mixing member 16 and the mixing member driving devices 17 can follow displacements of the pressing member 4 in the Z-direction.

The mixing member driving devices 17 may support the mixing member 16 via known lift mechanisms such as pistons so that the mixing member 16 can ascend and descend (can move in the Z-direction). As a consequence, the position of the mixing member 16 in the Z-direction can be adjusted independently from movements of the pressing member 4, for example, under the control of the control unit 13.

### <Mixing Method for Culture Fluid>

Using FIG. 11 further, a description will next be made about a mixing method for a culture fluid in the present embodiment which is not part of the present invention.

Described specifically, the mixing method of the present embodiment for the culture fluid is primarily characterized by placing, on the placement surface, the flexible culture vessel FP with cells C and culture fluid filled therein, pressing the flexible culture vessel FP with the pressing member 4, and then inserting and moving the mixing member 16 between the pressing member 4 and the flexible culture vessel FP with the outer surface, which is in contact with the placement surface, of the flexible culture vessel FP being kept deformed under the pressing by the pressing member 4.

Firstly, an initial position of the mixing member 16 is depicted in FIG. 11(a). As depicted in the figure, the mixing member 16 stands ready at the initial position, which corresponds to an end portion of the pressing member 4 or the placement surface, in an initial stage in which the pressing of the flexible culture vessel FP by the pressing member 4 is performed and aggregates C' begin to be formed in the flexible culture vessel FP.

However, the above-described initial position is not limited to the end portion of the pressing member 4 or the placement surface, and may be another position, for example, an end portion of the flexible culture vessel FP insofar as the internal pressure of the flexible culture vessel FP does not vary.

As depicted in FIG. 11(b) and FIG. 11(c) after a predetermined time has elapsed since the formation of the aggregates C' in the flexible culture vessel FP, the control unit 13 controls the mixing member driving devices 17 such that control is performed to move the mixing member 16 in an inserting and moving direction (the Y-direction). As a consequence, with the pressing of the flexible culture vessel FP by the pressing member 4 being maintained, the mixing member 16 is allowed to slide in and is inserted and moved between the pressing member 4 and the flexible culture vessel FP. The moving speed of the mixing member 16 in the inserting and moving direction may be set suitably, for example, according to the thickness of the flexible culture vessel FP, and may preferably be a relatively low speed (for example, from 1 to 5 mm/second). Instead of setting the moving speed of the mixing member 16 constant in the inserting and moving direction, the moving speed may be varied intermittently, for example, between 1 mm/second and 2 mm/second.

In the present embodiment which is not part of the present invention, following the above-described insertion (movement) of the mixing member 16, a localized flow of the culture fluid occurs in the flexible culture vessel FP at only a region around its contact with the mixing member 16, and this localized flow of the culture fluid shifts in the inserting and moving direction (the Y-direction) as the mixing member 16 moves. As this flow of the culture fluid is localized, no effect is given to the aggregates C' settled on the bottom of the flexible culture vessel FP.

As a consequence, the aggregates C' which exist in the recessed portions (wells) formed in the flexible culture vessel FP are prevented from being unevenly dispersed, and at the same time, the culture fluid which exists at an upper part in the flexible culture vessel FP can be efficiently mixed. In other words, the culture fluid can be gently mixed in the flexible culture vessel FP while the aggregates C' are kept settled.

The control unit 13 may adjust the pressing by the pressing member 4 such that the pressure applied to the flexible culture vessel FP remains substantially constant while the mixing member 16 is inserted and moved between the pressing member 4 and the flexible culture vessel FP. In other words, the control unit 13 may adjust the pressing by the pressing member 4 such that the internal pressure of the flexible culture vessel FP does not change before and after the mixing member 16 is inserted and moved between the pressing member 4 and the flexible culture vessel FP.

As a matter of fact, the internal pressure of the flexible culture vessel FP changes by the insertion and movement of the mixing member 16. However, by lifting the pressing member 4 a little by the control unit 13 (moving the pressing member 4 away from the placement surface), for example, the above-described rise in the internal pressure by the mixing member 16 can be offset.

As a consequence, the above-described mixing operation by the mixing member 16 can be performed without giving unnecessary effects to the aggregates C' in the flexible culture vessel FP.

As the mixing member 16, one having the shape that is rectangular in cross-section and has been chamfered in the round shape at the four corners thereof is used in the present embodiment, but the mixing member 16 is not limited to such a shape.

As depicted in FIG. 12(a), for example, one having a shape that is rectangular in cross-section and has been chamfered in a round shape at only corners on the side of a lower surface thereof, where the mixing member 16 comes into contact with the flexible culture vessel FP, may also be used.

Further, as depicted in FIG. 12(b), one having a cylindrical shape that is circular or elliptical in cross-section and extends in the X-direction may also be used.

Furthermore, as depicted in FIG. 12(c), one having a shape that is semicircular or semielliptical in cross-section and has an upper surface, which comes into contact with the pressing member 4 and is formed as a planer surface, may also be used.

The mixing member 16 in the present embodiment is disposed so that it extends perpendicularly relative to the inserting and moving direction (Y-direction), but is not limited to such a direction.

As depicted in FIG. 13, for example, the mixing member 16 may be disposed so that it is obliquely inclined relative to its inserting and moving direction of the mixing member 16.

Described more specifically, as depicted in FIG. 13(a), the mixing member 16 may have the shape that its center in the X-direction projects in the Y-direction (which may also be called "a boomerang shape" or "an inverted shallow V-shape"). As a consequence, it is possible to reduce a friction to be produced between the mixing member 16 and the flexible culture vessel FP in association with the insertion and movement of the mixing member 16. In addition, as depicted in FIG. 13(b), the positions of the mixing member driving devices 17 in the Y-direction disposed at opposite ends of the pressing member 4 may be shifted relative to each other so that the mixing member 16 is inclined relative to the X-direction. In this mode, it is also possible to reduce a friction to be produced between the mixing member 16 and the flexible culture vessel FP in association with the insertion and movement of the mixing member 16.

The mixing unit for the culture fluid in the present embodiment described above may be incorporated, for example, in the above-described cell culture apparatus. In addition, the mixing method for the culture fluid in the present embodiment may be incorporated, for example, in the above-described cell culture method.

In addition, in the present embodiment, the mixing member 16 is inserted and moved between the pressing member 4 and the flexible culture vessel FP with the outer surface of the flexible culture vessel FP, the outer surface being in contact with the placement surface, being kept deformed under the pressing by the pressing member 4, but the present invention is not limited to such a mode. Described specifically, the mixing member 16 may be allowed to slide and move on the upper surface of the flexible culture vessel FP with the outer surface, which is on the side of the placement surface, of the flexible culture vessel FP being kept deformed without using the pressing member 4, for example, as in the second embodiment and the fourth embodiment. In other words, the mixing member 16 may be slidingly moved on the upper surface of at least the flexible culture vessel FP with the flexible culture vessel FP being kept deformed at its outer surface on the side of the placement surface by applying an external force.

### <<Modification1>>

FIG. 14 is a front view depicting a modification of a cell culture apparatus 1, which can be applied to the individual embodiments described above. A control unit 13 in the cell culture apparatus 1 according to the modification is provided with a function to control pressure applying devices 5 so that the pressing member 4 is rocked about the X-axis. Described more specifically, the control unit 13 in the present modification changes the spatial orientation of the pressing member 4 by shifting the advance-retract cycles of the pressure applying devices 5a and 5b, which are connected to the pressing member 4, relative to each other.

After a pressing force is applied to the flexible culture vessel FP placed on the placement surface of the stage 2 by the pressing member 4, the control unit 13 then controls the pressure applying devices 5 to perform control so that the pressing member 4 is rocked (swung) in a small range about the X-axis while maintaining the pressing.

According to modification 1, by swinging the pressing member 4 about the X-axis, the culture fluid in the flexible culture vessel FP is mixed, whereby the culture fluid and permeated gas in the flexible culture vessel FP are effectively mixed together.

In the present modification, the description has been made of the example in which the pressing member 4 is swung about the X-axis. However, the pressing member 4 may be swung about the Y-axis, or may be swung about a desired axis other than the X-axis and Y-axis.

As described above, according to the modification 1, even relatively small swings of the pressing member 4 can produce large movements of the culture fluid in the flexible culture vessel FP. The modification 1 is, therefore, considered to be suited when such vigorous mixing (suspension) as swirling the cells C in the flexible culture vessel FP is conducted.

Accordingly, the modification 1 is not only limited to a cell culture apparatus but is also effective as a mixing method and mixing unit which is not part of the present invention. The mixing method for the culture fluid is characterized by pressing the flexible culture vessel FP, in which cells C and the culture fluid are filled, by the pressing member 4 with the flexible culture vessel FP being kept placed on the placement surface, and swinging the pressing member 4 to perform mixing of the culture fluid with the outer surface, which is in contact with the placement surface, of the flexible culture vessel FP being kept deformed under the pressing by the pressing member 4. On the other hand, the mixing unit for the culture fluid is characterized by including the placement surface that exert a deformation on the outer surface of the flexible culture vessel FP with cells C and the culture fluid filled therein, the pressing member 4 that applies a pressure to the flexible culture vessel FP placed on the placement surface, and the control unit 13 that performs control to swing the pressing member with the pressure being kept applied onto the flexible culture vessel FP by the pressing member 4.

### <<Modification 2>>

FIG. 15 depicts a perspective view and a cross-sectional view illustrating a stage in a cell culture apparatus in modification 2.

As depicted in FIG. 15(a), a stage 2''' in the present modification includes a placement surface 2'''a, and depressions 2'''b formed by tapered walls 2'''c. There are five depressions 2'''b in the present modification, but the number of depression(s) 2'''b can be a desired number other than five.

As depicted in FIG. 15(b), each tapered wall 2'''c is provided such that its diameter gradually increases toward the placement surface 2'''a.

According to the modification 2, the boundary between the placement surface 2'''a and each depression 2'''b takes an obtuse angle by the tapered wall 2'''c, so that the flexible culture vessel FP is prevented, for example, from being carelessly damage upon placing the flexible culture vessel FP on the stage 2'''.

### [Industrial Applicability]

The present invention can be suitably used, for example, when cells are mass-cultured while forming cell aggregates especially in a cell culture process.

### [Reference Signs List]

1 Cell culture apparatus
2, 2', 2", 2''' Stage
3 Stage supporting post
4 Pressing member
5 Pressure applying device
6 Vessel connection port
7 Observation device
8 Lighting device
9 Medium supply tank
10 Medium collection tank
11 Frame
12 Stage device
12a XY stage
12b Z stage
13 Control unit
14 Negative pressure source
15 Pressure regulator
16 Mixing member
17 Mixing member driving device
U Air-conditioning unit
FP Flexible culture vessel
C Cell

## Claims

1. A cell culture method comprising:
applying a pressure by pressing a pressing member (4) onto a flexible culture vessel (FP) filled with cells (C) and a culture fluid and placed on a placement surface (2a) in which a plurality of depressions (2b) are formed; and
culturing the cells in the flexible culture vessel with the flexible culture vessel being kept deformed at an outer surface (FPs) thereof, the outer surface being in contact with the placement surface, under the application of the pressure, so that a plurality of recessed portions are formed in and on the outer surface and then the cells gather in the recessed portions.

2. The cell culture method according claim 1, wherein
the application of the pressure is released during culture of the cells so that the outer surface of the flexible culture vessel, the outer surface being in contact with the placement surface, is planarized.

3. Use of a cell culture apparatus (20) for exerting a deformation on an outer surface of a flexible culture vessel in which cells and a culture fluid are filled, the cell culture apparatus (20) comprising:
a placement surface in which a plurality of depressions (2b) are formed; and
a pressing lid (26, 33) disposed opposite the placement surface and configured to be movable between a first position, where the pressing lid can hold the flexible culture vessel between the pressing lid and the placement surface while pressing the flexible culture vessel, and a second position, where at least a part of the pressing lid is more remote relative to the placement surface than at the first position.

4. A cell culture apparatus (1) comprising:
a placement surface (2a) configured to exert a deformation on an outer surface (FPs) of a flexible culture vessel (FP) in which cells and a culture fluid are filled, a plurality of depressions being formed in the placement surface; and
a control unit programmed to perform control to deform the outer surface, which is in contact with the placement surface, of the flexible culture vessel by applying a pressure onto the flexible culture vessel after placement of the flexible culture vessel on the placement surface by pressing a pressing member (4) onto the flexible culture vessel so that a plurality of recessed portions are formed in and on the outer surface.

5. The cell culture apparatus according to claim 4, further comprising:
an observation device (7) configured to observe an interior of the flexible culture vessel with the pressure being applied thereonto.

6. The cell culture apparatus according to claim 5, wherein
the observation device is configured to observe the interior of the flexible culture vessel through the placement surface.

7. The cell culture apparatus according to claim 5 or 6, further comprising:
a stage device (12) configured to move the observation device in at least a direction parallel to the placement surface.

8. The cell culture apparatus according to any one of claims 5 to 7, wherein
a plurality of observation devices are disposed for the placement surface.

## Patentansprüche

1. Zellkulturverfahren, aufweisend:
Ausüben eines Drucks durch Pressen eines Presselements (4) auf ein flexibles Kulturgefäß (FP), das mit Zellen (C) und einem Kulturfluid gefüllt ist und auf einer Ablagefläche (2a) abgelegt ist, in der mehrere Vertiefungen (2b) ausgebildet sind; und
Kultivieren der Zellen in dem flexiblen Kulturgefäß, wobei das flexible Kulturgefäß an einer Außenfläche (FPs) davon verformt gehalten wird, wobei die Außenfläche unter Aufbringung des Drucks in Kontakt mit der Ablagefläche gehalten wird, so dass mehrere vertiefte Abschnitte in und auf der Außenfläche gebildet werden und sich dann die Zellen in den vertieften Abschnitten sammeln.

2. Zellkulturverfahren nach Anspruch 1, wobei
die Aufbringung des Drucks während der Kultivierung der Zellen aufgehoben wird, so dass die Außenfläche des flexiblen Kulturgefäßes, die in Kontakt mit der Ablagefläche ist, planarisiert wird.

3. Verwendung einer Zellkulturvorrichtung (20) zum Ausüben einer Verformung auf eine Außenfläche eines flexiblen Kulturgefäßes, in das Zellen und ein Kulturfluid gefüllt sind, wobei die Zellkulturvorrichtung (20) aufweist:
eine Ablagefläche, in der mehrere Vertiefungen (2b) ausgebildet sind; und
einen Pressdeckel (26, 33), der gegenüberliegend der Ablagefläche angeordnet und so konfiguriert ist, dass er zwischen einer ersten Position, in welcher der Pressdeckel das flexible Kulturgefäß zwischen dem Pressdeckel und der Ablagefläche halten kann, während das flexible Kulturgefäß gepresst wird, und einer zweiten Position, in der zumindest ein Teil des Pressdeckels in Bezug auf die Ablagefläche weiter entfernt ist als in der ersten Position, bewegbar ist.

4. Zellkulturvorrichtung (1), aufweisend:
eine Ablagefläche (2a), die so konfiguriert ist, dass sie eine Verformung auf eine Außenfläche (FPs) eines flexiblen Kulturgefäßes (FP) ausübt, in das Zellen und ein Kulturfluid gefüllt sind, wobei in der Ablagefläche mehrere Vertiefungen ausgebildet sind; und
eine Steuerungseinheit, die so programmiert ist, dass sie eine Steuerung durchführt, um die Außenfläche des flexiblen Kulturgefäßes, die in Kontakt mit der Ablagefläche ist, zu verformen, indem nach dem Ablegen des flexiblen Kulturgefäßes auf der Ablagefläche durch Pressen eines Presselements (4) auf das flexible Kulturgefäß ein Druck auf das flexible Kulturgefäß ausgeübt wird, so dass in und auf der Außenfläche mehrere vertiefte Abschnitte gebildet werden.

5. Zellkulturvorrichtung nach Anspruch 4, ferner aufweisend:
eine Beobachtungsvorrichtung (7), die für das Beobachten eines Innenraums des flexiblen Kulturgefäßes konfiguriert ist, während der Druck darauf ausgeübt wird.

6. Zellkulturvorrichtung nach Anspruch 5, wobei
die Beobachtungsvorrichtung für das Beobachten des Innenraums des flexiblen Kulturgefäßes durch die Ablagefläche konfiguriert ist.

7. Zellkulturvorrichtung nach Anspruch 5 oder 6, ferner aufweisend:
eine Gestellvorrichtung (12), die so konfiguriert ist, dass sie die Beobachtungsvorrichtung in mindestens einer Richtung parallel zur Ablagefläche bewegt.

8. Zellkulturvorrichtung nach einem der Ansprüche 5 bis 7, wobei
mehrere Beobachtungsvorrichtungen für die Ablagefläche angeordnet sind.

## Revendications

1. Procédé de culture cellulaire comprenant :
l'application d'une pression en pressant un élément de pression (4) sur un récipient de culture souple (FP) rempli de cellules (C) et d'un fluide de culture et placé sur une surface de placement (2a) dans laquelle une pluralité d'enfoncements (2b) est formée ; et
la culture des cellules dans le récipient de culture souple, le récipient de culture souple étant maintenu déformé au niveau d'une surface externe (FPs) de celui-ci, la surface externe étant en contact avec la surface de placement, sous l'application de la pression, de sorte qu'une pluralité de parties évidées est formée dans et sur la surface externe, puis les cellules se rassemblent dans les parties évidées.

2. Procédé de culture cellulaire selon la revendication 1, où
l'application de la pression est relâchée pendant la culture des cellules de sorte que la surface externe du récipient de culture souple, la surface externe qui est en contact avec la surface de placement, est aplanie.

3. Utilisation d'un appareil de culture cellulaire (20) pour exercer une déformation sur une surface externe d'un récipient de culture souple que l'on a rempli de cellules et d'un fluide de culture, l'appareil de culture cellulaire (20) comprenant :
une surface de placement dans laquelle une pluralité d'enfoncements (2b) est formée ; et
un couvercle de pression (26, 33) disposé à l'opposé de la surface de placement et configuré pour être mobile entre une première position, où le couvercle de pression peut maintenir le récipient de culture souple entre le couvercle de pression et la surface de placement tout en pressant le récipient de culture souple, et une deuxième position, où au moins une partie du couvercle de pression est plus éloignée par rapport à la surface de placement que dans la première position.

4. Appareil de culture cellulaire (1) comprenant :
une surface de placement (2a) configurée pour exercer une déformation sur une surface externe (FPs) d'un récipient de culture souple (FP) que l'on a rempli de cellules et d'un fluide de culture, une pluralité d'enfoncements étant formée dans la surface de placement ; et
une unité de commande programmée pour effectuer une commande pour déformer la surface externe, qui est en contact avec la surface de placement, du récipient de culture souple en appliquant une pression sur le récipient de culture souple après le placement du récipient de culture souple sur la surface de placement en pressant un élément de pression (4) sur le récipient de culture souple de sorte qu'une pluralité de parties évidées est formée dans et sur la surface externe.

5. Appareil de culture cellulaire selon la revendication 4, comprenant en outre :
un dispositif d'observation (7) configuré pour observer un espace intérieur du récipient de culture souple lorsque la pression est appliquée sur celui-ci.

6. Procédé de culture cellulaire selon la revendication 5, où
le dispositif d'observation est configuré pour observer l'espace intérieur du récipient de culture souple à travers la surface de placement.

7. Appareil de culture cellulaire selon la revendication 5 ou 6, comprenant en outre :
un dispositif à étage (12) configuré pour déplacer le dispositif d'observation dans au moins une direction parallèle à la surface de placement.

8. Appareil de culture cellulaire selon l'une quelconque des revendications 5 à 7, où
une pluralité de dispositifs d'observation est disposée pour la surface de placement.
